# FASCICULE DE BREVET EUROPEEN

(11) **EP 0 618 966 B1**
(45) Date de publication et mention de la délivrance du brevet: **08.04.1998**
(21) Numéro de dépôt: 93902352.9
(22) Date de dépôt: 18.12.1992
(51) Int. Cl.: C12N 15/10, C12Q 1/68

(54) **PROCEDE DE PREPARATION D'ARN DOUBLE-BRIN, ET SES APPLICATIONS**
VERFAHREN ZUR VORBEREITUNG VON DOPPEL STRANGIGER RNA UND SEINE ANWENDUNGEN
METHOD FOR PREPARING DOUBLE STRANDED RNA, AND USE THEREOF

(30) Priorité: 18.12.1991 FR 9115710
(43) Date de publication de la demande: 12.10.1994
(73) Titulaire: CIS BIO INTERNATIONAL, 91400 Saclay (FR)
(72) Inventeur: LIVACHE, Thierry, F-38000 Grenoble (FR); FOUQUE, Brigitte, F-38170 Seyssinet (FR); TEOULE, Robert, F-38000 Grenoble (FR)
(74) Mandataire: Ores, Irène
(86) Numéro de dépôt international: FR9201209
(87) Numéro de publication internationale: WO9312229

(56) Documents cités:
- WO-A-90/14090
- WO-A-91/10746
- US-A- 4 766 072
- BIOCHEMISTRY INTERNATIONAL vol. 14, no. 6, Juin 1987, SYDNEY,AU pages 1015 - 1022 C.SADHU ET AL. 'In vitro synthesis of double stranded RNA and measurement of thermal stability: effect of base composition, formamide and ionic strength' cité dans la demande
- NUCLEIC ACIDS RESEARCH. vol. 17, 1989, ARLINGTON, VIRGINIA US page 10109 F.-U. GAST 'Multi-purpose vector for in vitro production of single- and double-stranded RNA'

## Description

La présente Invention est relative à un procédé de synthèse d'ARN double-brin, et à ses applications.

Les acides ribonucléiques double-brin sont naturellement rares on les retrouve en effet seulement dans certains microorganismes tels que des levures ou des virus. Jusqu'à une époque récente, l'ARN double-brin n'était considéré que comme une molécule présentant un intérêt essentiellement théorique, et dont les applications éventuelles ne concernaient que la recherche fondamentale.

Toutefois, il a été démontré que les ARNs double-brin peuvent, de façon transitoire, être impliqués dans des phénomènes de régulation d'expression, ainsi que dans l'initiation de la synthèse d'interféron par les cellules [(DECLERQ et al., Methods in Enzymology, 1981, 78, 291 et WU-LI, J. Biol. Chem., 1990, 265, 5470)] et qu'ils possédaient des propriétés anti-prolifératives, ce qui permet d'envisager également des applications thérapeutiques (AUBEL et al., Proc. Natl. Acad. Sci., USA 1991, 88, 906).

Il est donc important de pouvoir synthétiser de façon pratique de l'ARN double-brin (ARNds) de séquence et de longueur déterminées.

Cependant, l'obtention d'ARN double-brin est considérée comme difficile. Les quelques procédés décrits jusqu'à présent peuvent être répartis en trois catégories

### 1) Extraction d'ARN double-brin à partir de matériel biologique.

L'extraction de doubles brins d'ARN à partir de virus a été décrite, par exemple par BOCCARDO G. et al. (Double stranded RNA viruses, 1983, Bishop Eds. Elsevier, New-York) et plus récemment par DULIEU et al. (J. Virol. Methods, 1989, 24, 77-84).

La présence d'ARNds dans certaines levures a été démontrée par FRIED et al. (Proc. Natl. Acad. Sci. USA, 1978, 75, 4225) et par AL-HAKEEM et al. (Anal. Biochem., 1987, 163, 433-439).

Si l'extraction d'ARNds à partir de levures est intéressante sur le plan quantitatif, les séquences et les longueurs des ARNds sont bien sûr imposées par le micro-organisme lui-même. C'est une bonne voie de production d'ARNds en tant que matière première, mais qui ne permet en aucun cas la synthèse d'une séquence prédéterminée.

### 2) Hybridation de 2 ARN simple-brin

La synthèse d'ARNds par hybridation de deux ARN simple-brin complémentaires a été décrite par SADHER et al., (Biochem. Int., 1987, 14, 1015). Chaque chaîne d'ARN est alors synthétisée par transcription in vitro d'un plasmide recombinant contenant, en aval d'une séquence promotrice d'une ARN polymérase ADN dépendante, la séquence d'ADN à transcrire. Les ARN simple-brin sont ensuite purifiés et quantifiés puis réunis pour former par hybridation un double-brin d'ARN.

Plus récemment, BHATTACHARYYA (Nature, 1990, 343,484) utilise la synthèse d'ARN décrite par MILLIGAN (Nucleic Acids Res., 1987, 21, 8783) consistant en la transcription d'une matrice d'ADN synthétique en ARN. Les ARN simple-brin sont ensuite réunis et hybridés l'un sur l'autre.

Ces techniques sont relativement longues et malaisées à mettre en oeuvre, car elles imposent la préparation de deux ADN-matrices recombinants ou synthétiques et la purification des deux brins ARN produits préalablement à l'hybridation.

### 3) Production d'ARN double-brin homopolymérique

J. YANO et al. (Demande de Brevet Français 2 617 403) décrivent un procédé de préparation d'ARN double-brin de longueur définie, mais de séquence répétitive ou homopolymérique. En aucun cas, cette technique n'est applicable à la synthèse d'ARN de séquence plus complexe.

Or, les Inventeurs ont maintenant mis au point un procédé permettant la synthèse d'ARN double-brin à partir d'une matrice d'ADN de séquence donnée. Ils ont en effet constaté que si l'on procédait, dans des conditions déterminées, et dans un même compartiment réactionnel, à la transcription simultanée des deux brins complémentaires d'une séquence d'ADN, les deux transcrits formés s'hybridaient immédiatement entre eux, donnant ainsi naissance à un ARN double-brin.

La présente Invention a pour objet un procédé de préparation d'ARN double-brin, lequel procédé est caractérisé en ce que l'on procède à la transcription simultanée des deux brins complémentaires d'une séquence d'ADN présents dans un même mélange réactionnel. Dans ce but, la séquence à transcrire sur chacun desdits brins d'ADN est placée sous contrôle d'une séquence promoteur.

On entend par promoteur, toute séquence double brin d'ADN comprenant un site de fixation reconnu par une ARN polymérase-ADN dépendante. La fixation de l'ARN polymérase à cette séquence promoteur permet l'initiation de la transcription.

Parmi les séquences promoteur qui sont utilisables dans le cadre de la présente Invention, on citera par exemple les séquences reconnues par les ARN polymérases des phages T7, T3 ou SP6. Ceci ne représente toutefois pas une limitation, car il paraîtra clairement à l'homme du métier que toute séquence promoteur identifiée comme telle, et pour laquelle on dispose de l'ARN polymérase correspondante, est a priori utilisable.

Selon un mode de mise en oeuvre préféré de la présente Invention, les deux brins d'ADN destinés à être transcrits appartiennent à 2 duplex différents, qu'ils forment chacun avec un brin d'ADN de séquence au moins partiellement complémentaire.

Selon un autre mode de mise en oeuvre préféré de la présente Invention, les deux brins d'ADN destinés à être transcrits sont associés en un même duplex.

Dans ce mode de mise en oeuvre, la séquence d'ADN à transcrire en ARN double brin est encadrée de deux promoteurs, l'un contrôlant la transcription de l'un des brins, et l'autres celle du brin complémentaire. Ces deux promoteurs peuvent être identiques ou différents.

Les Inventeurs ont en effet constaté qu'un duplex d'ADN muni à chaque extrémité d'une séquence promotrice, peut engendrer directement des ARN de longueur définie et pouvant s'apparier deux à deux pour former un ARN double-brin.

Ceci est très surprenant quant on considère le mode d'action des ARN polymérases l'enzyme se fixe sur la séquence promotrice et se déplace sur la matrice d'ADN en synthétisant le brin d'ARN complémentaire. Il était jusqu'à présent supposé que si l'ADN matrice comportait deux séquences promotrices, le cheminement des enzymes, allant en sens inverse, était perturbé, aboutissant à de séquences incomplètes, et donc d'appariement difficile sinon impossible.

Or, les Inventeurs ont constaté que, contrairement à ce que l'on supposait précédemment, il est possible de transcrire simultanément et entièrement les deux brins complémentaires d'un duplex d'ADN ; l'hybridation des deux chaînes complémentaires d'ARN formées est alors immédiate.

Dans ces conditions, le rendement de la synthèse d'ADN double-brin est meilleur que celui d'un simple-brin puisque l'ARN formé sous forme de duplex est plus inerte que l'ARN simple-brin vis à vis de la matrice d'ADN et des nucléases.

Différentes méthodes pour réaliser des double-brins d'ADN matrice pourvus de promoteurs peuvent être utilisées.

A titre d'exemple, on citera les 3 méthodes suivantes
- Le double-brin matrice peut être construit par hybridation directe de deux oligonucléotides de séquences complémentaires, issus de synthèse chimique. Chaque oligonucléotide porte à chaque extrémité la séquence d'un promoteur spécifique d'une ARN polymérase, de préférence celle d'une ARN polymérase issue de phage, telle que l'ARN Polymérase du phage T7 (de telles séquences ont été décrites, par exemple, par CHAMBERLIN, The enzymes, 1982, vol XV, Academic Press).

Ces deux ADN simple-brin sont ajoutés en quantités équimolaires puis hybridés l'un avec l'autre. On obtient ainsi un double-brin d'ADN qui, dans le cas d'oligonucléotides de synthèse, peut avoir une longueur comprise préférentiellement entre 40 et 100 paires de bases, et qui est pourvu à chaque extrémité d'une séquence promoteur.
- Le double-brin d'ADN peut aussi être obtenu par hybridation partielle de deux oligonucléotides sur un court fragment de l'extrémité 3' ; puis par allongement des extrémités 3' par une ADN polymérase. Cette technique permet d'obtenir des matrices d'ADN de longueur supérieure et de coût inférieur à celles décrites précédemment.
- La troisième méthode consiste à intégrer des amorces portant en 5' des séquences promotrices d'au moins une ARN polymérase dans l'ADN à transcrire par un procédé d'amplification tel que la PCR (Polymerase Chain Reaction), la LCR (Ligase Chain Reaction, la NASBA (Nucleic Acid Sequence - Based Amplification), etc ... (voir pour revue RICHARD, Curr. Op. Biotec., 1991, 2, 76-85) . On obtient ainsi en fin de réaction un ADN double-brin de longueur comprise préférentiellement entre 50 et 1000 paires de bases et muni à chaque extrémité d'une séquence promotrice d'une ARN polymérase.

Le procédé PCR est décrit par SAIKI (Science, 1985, 230, 1350 et Science, 1988, 239, 487). L'intégration d'un promoteur par la PCR est décrit par MURAKAWA (DNA, 1988, 7, 287) et dans les brevets WO 89/07149 (SOMMER) et WO 89/01050 (BURG), qui décrivent les procédés de synthèse d'ARN simple-brin.

Pour la mise en oeuvre du procédé conforme à l'Invention, les matrices d'ADN ainsi obtenues sont incubées en présence de l'ARN polymérase (ou des ARN polymérases) reconnaissant le ou les promoteurs portés par ces matrices, et de ribonucléosides triphosphate. Les conditions de réaction sont déterminées en fonction du type d'ARN polymérase choisie.

Il est particulièrement avantageux d'utiliser une faible concentration d'enzyme entre 10 et 50 unités et une concentration élevée de ribonucléosides. triphosphates (entre 1 et 5 mM).

Par exemple, dans le cas de l'ARN polymérase du phage T7, on utilise préférentiellement 20 unités d'enzyme, et 2,5 mM de chaque ribonucléoside triphosphate.

Dans ces conditions, les deux chaînes complémentaires d'ARN formées s'hybrident instantanément pour former un ARN double-brin plus stable et plus inerte qu'un ARN simple-brin. Chaque ADN matrice engendre au moins une centaine de chaînes d'ARN.

Les ARN double-brin obtenus à partir d'une matrice d'ADN comprenant un promoteur sur chaque brin se présentent comme la copie en ARN de l'ADN matrice, dont les extrémités 3' sont constituées par un simple-brin de séquence complémentaire à celle du promoteur.

Tous les duplex-matrices d'ADN peuvent éventuellement être liés à un support ; cette fixation peut par exemple être effectuée par rajout sur l'extrémité 5' d'au moins un des oligonucléotides, d'un ligand tel que la biotine, qui peut être fixé sur un support recouvert d'avidine. Cette fixation permet non seulement une purification beaucoup plus rapide de l'ARN double-brin synthétisé, mais aussi une réutilisation possible de la matrice fixée sur le support.

L'ARN double-brin ainsi obtenu conformément à l'Invention peut être soit séparé de l'ADN et éventuellement purifié (cette opération est simplifiée si la matrice d'ADN est fixée sur un support), soit utilisé ou quantifié directement si on utilise cette technique comme outil analytique.

Le procédé conforme à l'Invention, de par sa simplicité de mise en oeuvre, permet de nombreuses applications.

Il permet en effet d'obtenir, en une seule étape, une quantité importante de fragments d'ARN double-brin, de longueur et de séquence déterminée, ce que ne permettaient pas les procédés de l'art antérieur.

Les fragments ainsi obtenus peuvent bien entendu, être utilisés dans les diverses applications relevant de la recherche fondamentale, ou de la thérapeutique, préconisées dans l'art antérieur (Cf par exemple les publications de DECLERQ, WU-LI et AUBEL. précitées).

En outre, le procédé de synthèse d'ARNs double-brin conforme à l'Invention peut être utilisé pour le diagnostic, et en particulier pour la détection de séquences cible d'acide nucléique dans un échantillon biologique. Au sens de la présente Invention,
on désigne par séquence cible, toute séquence d'acide nucléique dont on souhaite détecter la présence dans un échantillon à analyser.

De très nombreuses méthodes de diagnostic font appel à la détection d'une séquence spécifique d'acide nucléique dans un échantillon biologique.

Les premiers procédés de détection qui ont été proposés font appel à des sondes constituées par des fragments d'acide nucléique simple-brins complémentaires de la séquence cible à détecter. Ces sondes sont en outre marquées, ce qui permet la visualisation directe du produit d'hybridation séquence cible/sonde.

Toutefois, cette technique autorise seulement la détection de séquences représentées en un nombre d'exemplaires suffisamment important pour permettre l'obtention d'un signal d'hybridation visible.

Il a donc été proposé, pour améliorer la détection d'une séquence cible, d'augmenter le nombre de copies de celle-ci.

Les techniques actuellement le plus employées dans ce but sont basées sur le principe de l'amplification en chaîne par polymérase, ou PCR (Polymerase Chain Reaction). Cette technique permet l'amplification sélective d'une séquence d'ADN double-brin.

Le principe de la PCR, qui est bien connu, sera seulement brièvement rappelé ci-après l'amplification d'une séquence cible se fait par une succession de cycles, chaque cycle comprenant
- la dénaturation de l'ADN comprenant la séquence à amplifier ;
- l'hybridation d'amorces encadrant ladite séquence à l'extrémité 3' de chacun des brins à amplifier ;
- l'élongation en 3' des amorces par une ADN polymérase.
- la dénaturation des duplex d'ADN résultant de l'élongation des amorces, ce qui inaugure un nouveau cycle.

La séquence cible est ainsi, en théorie, amplifiée exponentiellement ; en pratique, après la phase d'amplification exponentielle, on parvient à une phase de plateau, et on obtient environ 10⁵ copies au bout de 30 cycles d'amplification.

Les fragments d'ADN double-brin résultant de l'amplification de la séquence cible peuvent ensuite être identifiés, soit par hybridation avec une sonde spécifique marquée, soit par visualisation directe après électrophorèse sur gel d'agarose, en présence d'un agent intercalant tel que le bromure d'éthidium, etc...

Toutefois, les techniques d'amplification par PCR posent certains problèmes qui compliquent leur mise en oeuvre, en particulier pour l'analyse d'un grand nombre d'échantillons.

Les techniques de détection faisant appel à l'hybridation d'une sonde marquée sont généralement considérées comme très spécifiques car la sonde est choisie pour ne reconnaître que la séquence cible. Elles présentent toutefois l'inconvénient de nécessiter l'utilisation de sondes marquées dont le coût est élevé, et dont l'hybridation nécessite plusieurs étapes complémentaires qui rallongent le temps de manipulation.

Dans le cadre d'analyses en série, il est beaucoup plus rapide, et moins coûteux, de procéder à une visualisation directe des fragments amplifiés. Toutefois, cette visualisation directe fait intervenir une molécule intercalante qui ne se fixe pas uniquement sur les fragments amplifiés, mais sur tous les acides nucléiques présents dans le mélange réactionnel, ce qui produit un bruit de fond important.

L'Invention apporte maintenant une solution à ce problème, en proposant la détection de copies sous forme d'ARN double-brin, des fragments d'ADN résultant de l'amplification de la séquence cible.

Grâce au procédé conforme à l'Invention, ces copies sous forme d'ARN double-brin peuvent facilement être obtenues.

Il a déjà été proposé (par exemple par la Demande Européenne 397 269, et par la Demande PCT 88/10315) pour améliorer la détection d'une séquence cible, de synthétiser de l'ARN simple-brin, à partir d'une copie d'ADN double-brin de cette séquence cible, dans l'un des brins de laquelle a été inséré un promoteur, et éventuellement d'amplifier les transcrits simple-brin obtenus, par l'action de la réplicase Qβ.

Toutefois, la détection des ARNs simple-brin ainsi obtenus n'est réalisée que par hybridation de sondes spécifiques, et d'autre part, les ARN simple-brin sont facilement dégradés par les nucléases. En revanche, les ARN double-brin obtenus conformément à l'Invention ne présentent pas ces inconvénients.

L'utilisation du procédé d'obtention d'ARN double-brin conforme à l'Invention pour la détection de séquences spécifiques dans un échantillon biologique complète avantageusement une technique de type PCR dont elle permet d'augmenter la spécificité et la sensibilité.

Par exemple, un ADN matrice muni de promoteurs peut être réalisé de façon spécifique, à partir d'un échantillon d'acide nucléique, par une technique telle que la PCR, en utilisant des amorces contenant des séquences promoteur pour l'amplification de la séquence cible. Seul un échantillon positif contenant la séquence-cible permettra la synthèse de la matrice d'ADN. Cette matrice est ensuite transcrite en au moins une centaine de copies d'ARN double-brin par une (ou des) ARN polymérase(s). Cet ARN double-brin, de longueur et de séquence définie par la matrice, et donc par l'acide nucléique cible, peut être alors quantifié par des moyens de dosage adéquats des acides nucléiques.

Il faut bien noter que la présence d'un ARN double-brin dans le milieu implique la présence de l'acide nucléique cible dans l'échantillon ; en effet, si l'échantillon est négatif, l'ADN matrice ne se forme pas et les promoteurs restent alors sous forme de simples brins d'ADN, qui ne peuvent pas être reconnus par l'ARN polymérase.

La détection ou le dosage de l'ARN double-brin formé peut être effectuée par tout moyen approprié, tel que l'hybridation avec une sonde d'acide nucléique spécifique de la séquence à détecter ; il est particulièrement avantageux de procéder à cette détection en milieu homogène, par mesure d'un signal induit par l'interaction spécifique d'une molécule intercalante avec la double chaîne d'ARN. Cette révélation ne nécessite pas l'utilisation d'une sonde spécifique, ni celle d'un support. Elle est donc rapide et peu coûteuse.

Divers agents intercalants peuvent être utilisés pour détecter l'ARN double brin obtenu par le procédé conforme à l'Invention. On préféra ceux qui interagissent plus spécifiquement avec l'ARN qu'avec l'ADN. Les Inventeurs ont en particulier constaté que l'utilisation de l'homodimère d'éthidium ou de l'iodure de propionium comme agent intercalant permettait de détecter et de doser l'ARN obtenu, dans d'excellentes conditions de sensibilité et de spécificité.

Par rapport à une mesure directe de la fluorescence en présence d'un intercalant de la quantité d'ADN résultant d'une PCR, le procédé conforme à l'Invention présente les avantages suivants
- la transcription qui engendre au moins une centaine de copies de chaque double brin d'ADN multiplie le signal de fluorescence par un facteur égal ou supérieur à 100, et le rapport signal sur bruit est bien meilleur, puisque les amorces et les simples brins d'ADN résiduels qui ne peuvent être transcrits en ARN, n'interfèrent pas significativement avec la molécule intercalante.
- l'amplification terminale par transcription permet une meilleure quantification du procédé PCR réalisé en amont. En effet, cette méthode permet de réduire le nombre de cycles PCR préalables et donc de rester dans la phase d'amplification purement exponentielle de type (1+x)ⁿ de la PCR. D'autre part, l'amplification supplémentaire résultant de la transcription en ARNds selon le procédé conforme à l'Invention est linéaire ; en d'autres termes, le facteur d'amplification est constant, et ne dépend pas de la quantité d'ADN matrice présent dans le mélange réactionnel. On a donc un phénomène d'amplification globalement exponentiel puis linéaire ; l'analyse de cette fonction mathématique est beaucoup plus simple que dans le cas de la technique PCR classique dont la quantification est compliquée par l'apparition d'un plateau au delà de la zone exponentielle.

Cette meilleure régularité d'amplification associée à une révélation simple en une étape entraîne une meilleure précision de la mesure, et permet de la répéter aisément, ce qui facilite la mise en oeuvre de mesures quantitatives.

En outre, certains modes de mise en oeuvre préférés du procédé conforme à l'Invention permettent des perfectionnements particulièrement avantageux pour la détection de séquences cibles d'acide nucléique.

En particulier, il est possible, en mettant en oeuvre le procédé conforme à l'Invention, de synthétiser des ARNs qui n'ont une structure en double-brin que sur une partie de leur longueur et dont au moins l'une des extrémités est sous forme de simple-brin.

Ces ARNs peuvent être fixés à un support solide et/ou à une sonde de révélation par l'intermédiaire de leur partie simple-brin.

Ceci permet en particulier les applications suivantes :

Un ARN double brin comportant une ou deux extrémités simple brin peut être réalisé par transcription simultanée de deux duplex d'ADN (comportant chacun une séquence promotrice d'une ARN polymérase) dont une partie de la séquence est commune.

Dans ces conditions,l'ARN obtenu est sous forme double brin avec une ou deux extrémités de séquence simple brin. Cet hybride d'ARN qui est obtenu avec un coefficient d'amplification supérieur à 100 peut être hybridé sur un acide nucléique (acide nucléique récepteur) de séquence complémentaire à celle d'une des extrémités fixé sur un support solide.

L'acide nucléique récepteur (préférentiellement un oligonucléotide) est fixé sur le support de façon covalente ou non covalente. Des oligonucléotides de séquences différentes peuvent être fixés sur le même support sur des localisations différentes ; ainsi, plusieurs ADN cibles peuvent être transcrits dans le même flacon, et les transcrits détectés sur un support commun. La révélation est assurée, par exemple, par la fluorescence du double brin d'ARN engendrée par un composé intercalant exposé aux U.V.

Par ce moyen, plusieurs séquences cibles peuvent être traitées dans le même tube et détectées de façon spécifique par des acides nucléiques fixés sur un support.

La présente Invention sera mieux comprise à l'aide du complément de description qui va suivre, qui se réfère à des exemples de mise en oeuvre du procédé conforme à l'Invention.

### EXEMPLE 1 - APPLICATION ANALYTIQUE DETECTION DU VIRUS HPV 6/11 PAR FLUORESCENCE EN MILIEU HOMOGENE APRES SYNTHESE D'ARN DOUBLE-BRIN

L'ADN est détecté de façon spécifique par une procédure telle que celle décrite dans la Demande PCT WO90/15881. L'échantillon est d'abord amplifié par un couple d'amorces primaires, ce produit est ensuite fortement dilué, puis réamplifié à l'aide d'amorces incluant en 5' une séquence promotrice de l'ARN polymérase T7. La transcription en ARN double-brin est alors réalisée directement à partir du produit d'amplification et la fluorescence du produit final est mesurée après intercalation de bromure d'éthidium.

### 1. PCR primaire

Dix nanogrammes d'ADN de cellules, infectées ou non par le papillomavirus de type 6/11, sont amplifiés dans un volume de 50 µl en présence de 200 µM de dATP dCTP dTTP dGTP, 10 mM de Tris HCl pH 8,5 ; 50 mM KCl ; 1,5 mM MgCl₂ ; 0,01% de gélatine ; 2,5 U de Taq polymérase et de 10 pmoles d'oligonucléotides amorces.

La position dans HPV 6/11 et la séquence des amorces primaires utilisées sont les suivantes

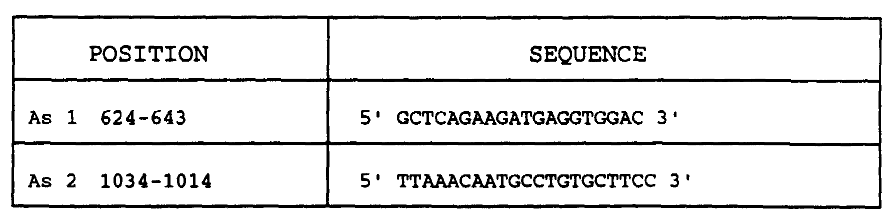

25 cycles de trois fois 1,5 min. (92°C, 55°C, 72°C) sont réalisés

### 2. Dilution intermédiaire au 1/200

2 µl de produit de la réaction de PCR primaire sont diluée dans 400 µl d'eau. 2 µl de cette solution sont utilisés pour l'amplification PCR secondaire.

### 3. PCR secondaire

Le même milieu réactionnel et les mêmes conditions que pour l'amplification primaire sont utilisées ; seules la composition et la quantité des amorces secondaires sont changées

La position dans HPV 6/11 et la séquence des amorces portant en 5' la séquence promotrice de la T7 RNA pol sont les suivantes

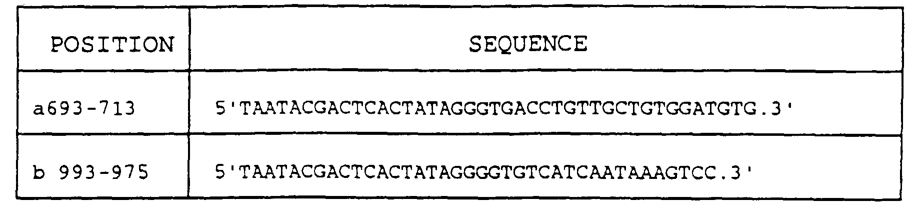

Quantité d'amorce utilisée : 2 pmoles pour 50 µl de milieu réactionnel (ou 1 pmole pour 25 µl).

15 cycles d'amplification sont effectués.

### 3. Réaction de transcription

La transcription en ARN double-brin est réalisée directement sur le produit amplifié ; on ajoute volume à volume, une solution contenant du Tris HCl 80 mM pH 8,0, NaCl 50 mM, MgC12 20 mM, spermidine 4 mM, DTT 10 mM, NTP (ribonucléosides triphosphate) 2,5 mM et 25 U de T7 RNA polymérase (BRL) dans H₂O DEPC stérile.

La réaction est conduite à 37°C durant 15 minutes.

### 4. Lecture par fluorescence au BET

Le produit transcrit est ajouté dans un milieu de mesure composé de Tris HCl 5 mM pH 7,6, NaCl 8 mM, EDTA 5 mM, BET (bromure d'éthidium) 0,5 µg/ml.

Les conditions de mesure de fluorescence sur un spectrofluorimètre Perkin-Elmer sont les suivantes longueur d'onde d'excitation 510 nm (sw 8 nm), d'émission 590 nm (sw 8nm), mesure en cuve plastique de 1 ml.

### 5. Résultats

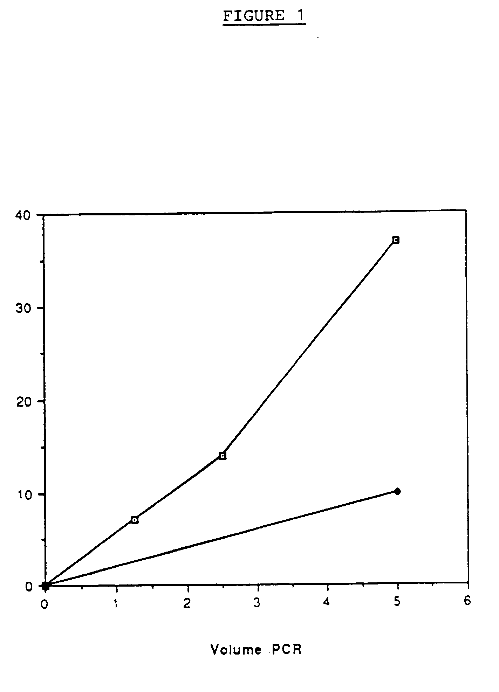

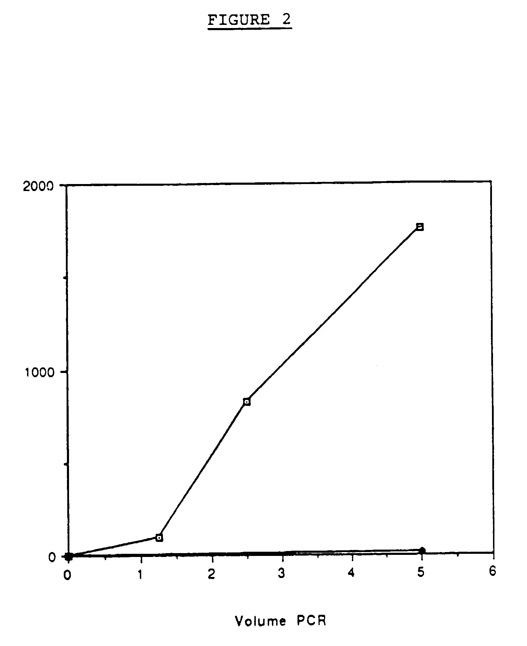

Deux techniques de fluorescence en milieu homogène appliquées, ont été comparées. Les résultats sont illustrés dans les Figures 1 et 2.

Figure 1 : Mesure directe de la fluorescence (360/450 nm) de l'ADN après amplification par PCR, par intercalation de H33 (HOECHST 33258) (0,5 µg/ml)
- □ = échantillon positif, ◆ = témoin négatif.

Figure 2 : Mesure par le BET après transcription en ARN double-brin selon la méthode conforme à l'Invention
- □ = échantillon positif, ◆ = témoin négatif.

Les résultats sont exprimés, pour une même prise d'essai de produit, par le pourcentage de fluorescence (en unités arbitraires).

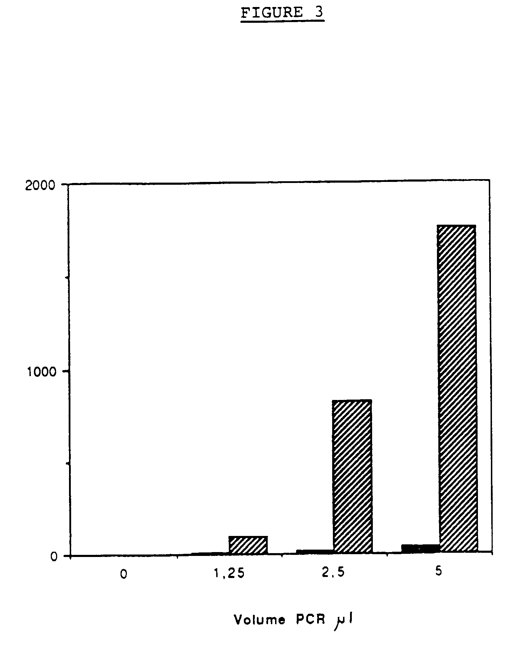

La figure 3 permet la comparaison entre l'intensité des signaux obtenus
- ■ directement après amplification par PCR (fluorescence du H33)
- □ après transcription en ARN double-brin, conformément à l'Invention (fluorescence du BET).

Pour un équivalent de 5 µl de produit PCR, on mesure une fluorescence de l'ADN par le H33 de 35 unités (contre 5 pour le négatif) alors que la fluorescence est de 1800 unités, pour une même prise d'essai après transcription en ARN double brin et mesure au BET (contre 12 pour le négatif) .

### EXEMPLE 2 - SYNTHESE PREPARATIVE D'UN ARN DOUBLE-BRIN PAR TRANSCRIPTION SUR SUPPORT

On prépare une matrice d'ADN double-brin, comprenant sur chaque brin un promoteur pour l'ARN polymérase, et un groupe biotine en 5' de l'un des brins. Cette matrice est ensuite fixée sur un support recouvert d'avidine et la transcription est réalisée directement sur ce support.

### 1. Préparation de la matrice d'ADN

La matrice préparée dans cet exemple représente un fragment du génome de HPV 6/11.

### a) A partir d'un échantillon biologique

La matrice est préparée par amplification d'un fragment d'ADN de HPV 6/11. Les amplifications primaire et secondaire sont menées selon le procédé décrit dans l'exemple 1, l'amorce T7a étant préalablement biotinylée en 5' par la méthode de ROGET et al; (Nucleic Acids Res., 1989, 17, 7643-7651). Si l'ADN de départ est suffisamment purifié, il est possible de préparer directement la matrice en procédant à une seule étape d'amplification, dans laquelle on utilise les amorces T7a (biotinylée en 5') et T7b.

### b) A partir d'ADN de synthèse

Le double brin d'ADN est réalisé par hybridation partielle des deux oligonucléotides bio-T₇O₁ et T₇O₂ ci-dessous, puis élongation des 2 brins

- bio-T7O1
5'  (biotine)                                    3'
TTTTAATACGAGTCACTATAGGGTACCGCTTAGCTAATGGACGCTAC

- T7O2
5'                                                    3'
TAATACGAGTCACTATAGGGGTAGCGTCCATTAGCTAAGCGGTA

### 2. Fabrication du support avidine

Un "coating" de BSA (Sérum albumine bovine) biotine puis d'avidine est réalisé dans un tube à ailettes Nunc en polystyrène (SAUVAIGO et al. Nucl. Acids Res. 1989,18, 3175-3183). Ce tube peut être conservé plusieurs mois à +4°C.

### 3. Fixation de la matrice d'ADN sur le support

Dix microlitres du produit PCR biotinylé synthétisé préalablement est fixé sur le support par incubation dans 100 µl de tampon Tris-HCl 0,05 M pH 9,2 NaCl 0,5 M 2 heures à 37°C. Le tube est ensuite rincé trois fois par ce même tampon en présence de 0,5% de SDS puis une fois par de l'eau stérile.

Ce support muni d'une matrice d'ADN peut être conservé à +4°C plusieurs semaines.

### 4. Transcription

La réaction de transcription de la matrice fixée sur le support en ARN double-brin est assurée par 50 U d'ARN polymérase de T7 (BRL) dans 100 µl de 0,04 M Tris HCl pH 8,0 ; 8 mM MgCl₂ ; 2 mM spermidine ; 25 mM NaCl ; 5 mM DTT et 2 mM de ATP, CTP, UTP, GTP.

La réaction est conduite à 37°C durant 30 minutes.

### 5. Isolement et contrôle de l'ARN synthétisé

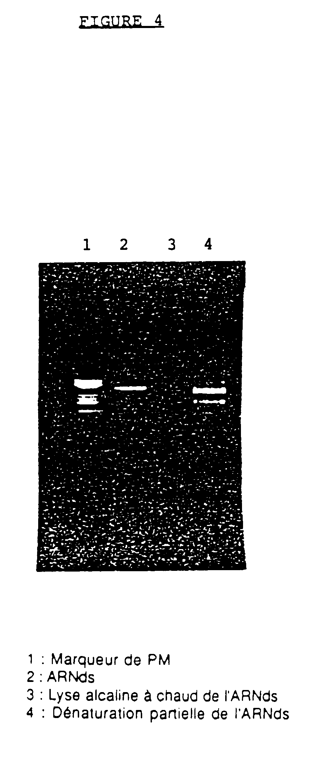

100 microlitres de milieu réactionnel de transcription sont récupérés et mélangés avec 400 µl d'éthanol froid. Les ARN sont ensuite précipités à -20°C puis isolés par centrifugation. Le culot obtenu est repris par de l'eau stérile puis quantifié par spectrométrie UV. L'intégrité de cet ARN double-brin est contrôlée par migration électrophorétique sur gel d'agarose. Les profils électrophorétiques sont représentés à la figure 4. La structure en double-brin est prouvée par une dénaturation thermique (chauffage à 100°C). La nature ribonucléotidique est confirmée par lyse alcaline à chaud.

Environ 50 microgrammes d'ARN (soit 1 unité de D.O à 260 nm) sont synthétisés lors de chaque réaction.

Le support rincé et stocké à +4°C peut être utilisé ultérieurement plusieurs fois, sans perte notable d'activité.

### EXEMPLE 3 - SYNTHESE D'UN ARN DOUBLE-BRIN COMPORTANT UNE EXTREMITE SIMPLE BRIN DE SEQUENCE SPECIFIQUE DE L'ADN CIBLE : DETECTION DU VIRUS HPV6-II

La procédure utilisée comprend la formation de 2 duplex d'ADN (chacun muni d'une séquence promotrice de la T7 RNA polymérase) par 2 réactions PCR conduites en parallèle sur chaque échantillon.

Ces 2 fractions sont réunies puis transcrites en ARN qui est sous forme partiellement double brin.

L'ARN obtenu comporte une partie double brin de 159 bases de long, suivie d'une partie simple brin de 141 bases. Ce produit est ensuite hybridé sur un oligonucléotide fixé sur une membrane de nylon puis détecté par fluorescence.

### 1. Formation des duplex d'ADN

Un échantillon d'ADN est réparti dans 2 tubes. Deux amplifications PCR sont alors réalisées de façon indépendante dans les conditions décrites dans l'exemple 1, grâce aux couples d'amorces suivants 
IT7a 693-713
5' 3'
TAATACGACTCACTATAGGGTGACCTGTTGCTGTGGATGTG
 
Ib' 852-830
5' 3'
TGTACCTGAATCGTCCGCCATC
 
IIa  693-713
5'                    3'
TGACCTGTTGCTGTGGATGTG
 
IIT7b 993-975
5' 3'
TAATACGACTCACTATAGGGGTGTCATCAATAAAGTCC
 30 cycles (92-55-72, 1,30) sont réalisés.

### 2. Transcription

Les 2 produits PCR sont mélangés volume à volume et 2 volumes de milieu de transcription contenant 25 U de T7RNA polymérase sont ajoutés (voir exemple 1).

La transcription est conduite durant 30 minutes à 42°C.

### 3. Détection du semi-duplex d'ARN sur support solide

Un oligonucléotide de séquence complémentaire à une partie de l'extrémité simple brin de l'ARN est fixé sur une membrane de nylon selon la technique décrite par SAIKI et al. Proc. Natl. Acad. Sci. USA, 86, 6230-6234 (1989).

Oligonucléotide fixé sur le support

HPV6/11 914-940
5'                                    3'
CAGGTACACAAATATCAGACGATGAGG

Le semi-duplex d'ARN est hybridé 1 heure sur ce support (SSPE 5X, SDS 0,1% à 55°C). La membrane est ensuite rincée dans le même milieu 5 minutes à 55°C puis rapidement dans un tampon phosphate 5 mM, pH 7,0.

La révélation est alors assurée par incubation (2 minutes) dans une solution à 0,5 µg/ml d'homodimère d'éthidium, dans du tampon phosphate 5 mM suivi d'un bref rinçage. Les spots fluorescents sont alors directement observés sous rayonnement à 260 nm et à 520 nm.

Les échantillons positifs présentent une nette fluorescence orangée alors que les négatifs ne se distinguent pas du bruit de fond.

## Revendications

1. Procédé de préparation d'ARN double-brin, lequel procédé est caractérisé en ce que l'on procède à la transcription simultanée des deux brins complémentaires d'une séquence d'ADN présents dans un même mélange réactionnel et à l'hybridation immédiate des deux transcrits obtenus.

2. Procédé selon la Revendication 1, caractérisé en ce que les deux brins d'ADN destinés à être transcrits sont sous contrôle d'une séquence promoteur reconnue par la même ARN polymérase.

3. Procédé selon la Revendication 1, caractérisé en ce que les deux brins d'ADN destinés à être transcrits sont sous contrôle de séquences promoteurs, reconnues par deux ARN polymérases différentes.

4. Procédé selon l'une quelconque des Revendications 1 à 3, caractérisé en ce que les deux brins d'ADN destinés à être transcrits appartiennent à deux duplex différents.

5. Procédé selon l'une quelconque des Revendications 1 à 3, caractérisé en ce que les deux brins d'ADN destinés à être transcrits sont associés dans un même duplex.

6. Procédé selon l'une quelconque des Revendications 1 à 5, caractérisé en ce que les brins d'ADN destinés à être transcrits sont fixés sur un support solide.

7. Procédé selon l'une quelconque des Revendications 1 à 6, caractérisé en ce qu'il comprend en outre une étape de détection et/ou de dosage de l'ARN double brin obtenu.

8. Méthode de détection et/ou de dosage d'une séquence cible d'ADN dans un échantillon biologique, laquelle méthode est caractérisée en ce qu'elle comprend une étape au cours de laquelle l'on procède à la transcription simultanée, de tout ou partie des deux brins complémentaires de ladite séquence cible, par un procédé selon l'une quelconque des Revendications 1 à 7.

9. Méthode selon la Revendication 8, caractérisée en ce qu'elle comprend préalablement à l'étape de transcription, au moins une étape au cours de laquelle l'on procède à l'amplification de la séquence cible.

10. Méthode selon l'une quelconque des Revendications 8 ou 9, caractérisée en ce qu'au moins un segment du produit de transcription de la séquence cible à détecter est sous forme d'ARN simple-brin, et en ce que ledit produit de transcription est fixé sur un support solide par l'intermédiaire d'un segment simple-brin.

11. Méthode selon l'une quelconque des Revendication 8 à 10, caractérisée en ce que plusieurs séquences cibles différentes sont transcrites et détectées simultanément dans un même échantillon.

## Patentansprüche

1. Verfahren zur Herstellung einer doppelsträngigen RNA, dadurch **gekennzeichnet**, daß eine gleichzeitige Transkription der beiden komplementären Stränge einer im Reaktionsgemisch vorhandenen DNA und eine sofortige Hybridisierung der beiden erhaltenen Transkripte erfolgt.

2. Verfahren nach Anspruch 1, dadurch **gekennzeichnet**, daß die beiden zu transkribierenden DNA-Stränge von einer Promotorsequenz, die von der gleichen RNA-Polymerase erkannt wird, kontrolliert werden.

3. Verfahren nach Anspruch 1, dadurch **gekennzeichnet**, daß die beiden zu transkribierenden DNA-Stränge von Promotorsequenzen, die von zwei verschiedenen RNA-Polymerasen erkannt werden, kontrolliert werden.

4. Verfahren nach einem der Ansprüche 1 bis 3, dadurch **gekennzeichnet**, daß die beiden zu transkribierenden DNA-Stränge auf zwei verschiedenen Duplexen liegen.

5. Verfahren nach einem der Ansprüche 1 bis 3, dadurch **gekennzeichnet**, daß die beiden zu transkribierenden DNA-Stränge auf dem gleichen Duplex assoziiert sind.

6. Verfahren nach einem der Ansprüche 1 bis 5, dadurch **gekennzeichnet**, daß die zu transkribierenden DNA-Stränge auf einem festen Träger fixiert sind.

7. Verfahren nach einem der Ansprüche 1 bis 6, dadurch **gekennzeichnet**, daß es zusätzlich eine Nachweisstufe und/oder eine Stufe zur quantitativen Bestimmung der erhaltenen doppelsträngigen RNA umfaßt.

8. Verfahren zum Nachweis und/oder zur quantitativen Bestimmung einer DNA-Zielsequenz in einer biologischen Probe, dadurch **gekennzeichnet**, daß es eine Stufe umfaßt, bei der eine gleichzeitige Transkription der gesamten beiden komplementären Stränge der Zielsequenz oder Teilen davon mit einem Verfahren nach einem der Ansprüche 1 bis 7 erfolgt.

9. Verfahren nach Anspruch 8, dadurch **gekennzeichnet**, daß es vor der Transkriptionsstufe wenigstens eine Stufe umfaßt, im Verlauf derer eine Amplifikation der Zielsequenz erfolgt.

10. Verfahren nach einem der Ansprüche 8 oder 9, dadurch **gekennzeichnet**, daß wenigstens ein Abschnitt des Transkriptionsproduktes der nachzuweisenden Zielsequenz in Form einzelsträngiger RNA vorliegt und daß das Transkriptionsprodukt über einen einzelsträngigen Abschnitt auf einem festen Träger fixiert ist.

11. Verfahren nach einem der Ansprüche 8 bis 10, dadurch **gekennzeichnet**, daß mehrere verschiedene Zielsequenzen gleichzeitig in der gleichen Probe transkribiert und nachgewiesen werden.

## Claims

1. A process for preparing double-stranded RNA, in which process the simultaneous transcription is carried out of the two complementary strands of a DNA sequence which are present in the same reaction mixture and the immediate hybridization is carried out of the two transcripts obtained.

2. The process as claimed in claim 1, wherein the two strands of DNA intended to be transcribed are under the control of a promoter sequence recognized by the same RNA polymerase.

3. The process as claimed in claim 1, wherein the two strands of DNA intended to be transcribed are under the control of promoter sequences recognized by two different RNA polymerases.

4. The process as claimed in any one of claims 1 to 3, wherein the two DNA strands intended to be transcribed belong to two different duplexes.

5. The process as claimed in any one of claims 1 to 3, wherein the two strands of DNA intended to be transcribed are combined in the same duplex.

6. The process as claimed in any one of claims 1 to 5, wherein the strands of DNA intended to be transcribed are attached to a solid support.

7. The process as claimed in any one of claims 1 to 6, which comprises, in addition, a step for the detection and/or assay of the double-stranded RNA obtained.

8. A method for the detection and/or assay of a target DNA sequence in a biological sample, which method comprises a step during which the simultaneous transcription of all or part of the two complementary strands of the said target sequence is carried out by a process as claimed in any one of claims 1 to 7.

9. The method as claimed in claim 8, which comprises, prior to the transcription step, at least one step during which the amplification of the target sequence is carried out.

10. The method as claimed in either of claims 8 or 9, wherein at least one segment of the product of transcription of the target sequence to be detected is in the form of single-stranded RNA, and wherein the said transcription product is attached onto a solid support via a single-stranded segment.

11. The method as claimed in any one of claims 8 to 10, wherein several different target sequences are transcribed and detected simultaneously in the same sample.
